# EUROPEAN PATENT APPLICATION

(11) **EP 1 435 520 A1**
(43) Date of publication of application: **07.07.2004**
(21) Application number: 02770208.3
(22) Date of filing: 20.09.2002
(51) Int. Cl.: G01N 33/53, G01N 37/00

(54) **SUPPORT FOR LIGAND IMMOBILIZATION**

(30) Priority: 21.09.2001 JP 2001288149
(71) Applicant: TAKARA BIO INC., Otsu-shi, Shiga 520-2193 (JP)
(72) Inventor: ASADA, Kiyozo, Koka-gun, Shiga 520-3333 (JP); IMOSE, Nobuyuki, Kyoto-shi, Kyoto 601-8448 (JP); TAKEDA, Osamu, Hikone-shi, Shiga 521-1124 (JP); ROKUSHIMA, Masatomo, Toyonaka-shi, Osaka 561-0822 (JP); KATO, Ikunoshin, Koga-gun, Shiga 529-1851 (JP)
(74) Representative: Schnappauf, Georg Dr.
(86) International application number: PCT/JP2002/009661
(87) International publication number: WO 2003/027674

(57) **Abstract**

A support for ligand immobilization coated with a polyanion which has been activated beforehand.

SEQUENCE LISTING

<110> TAKARA BIO INC.

<120> The substrate for ligand immobilization

<130> 663404

<150> JP 2001-288149
<151> 2001-09-21

<160> 40

<210> 1
<211> 20
<212> DNA
<213> Artificial Sequence

<220>
<223> Designed oligonucleotide primer for amplifying DNA fragment of
corresponding gene

<400> 1
ctgaaggtca ccctagctga    20

<210> 2
<211> 20
<212> DNA

<213> Artificial Sequence

<220>
<223> Designed oligonucleotide primer for amplifying DNA fragment of
corresponding gene

<400> 2
gtaggttcca gttgaaagag    20

<210> 3
<211> 20
<212> DNA
<213> Artificial Sequence

<220>
<223> Designed oligonucleotide primer for amplifying DNA fragment of
corresponding gene

<400> 3
tccatctcct ggtgttctgc    20

<210> 4
<211> 20
<212> DNA
<213> Artificial Sequence

<220>

<223> Designed oligonucleotide primer for amplifying DNA fragment of
corresponding gene

<400> 4
taaacaggat tattattagt    20

<210> 5
<211> 20
<212> DNA
<213> Artificial Sequence

<220>
<223> Designed oligonucleotide primer for amplifying DNA fragment of
corresponding gene

<400> 5
taatttactt tgcaagaaaa    20

<210> 6
<211> 20
<212> DNA
<213> Artificial Sequence

<220>
<223> Designed oligonucleotide primer for amplifying DNA fragment of
corresponding gene

<400> 6

ctggatatta ttcttgtagt    20

<210> 7

<211> 20

<212> DNA

<213> Artificial Sequence

<220>

<223> Designed oligonucleotide primer for amplifying DNA fragment of

corresponding gene

<400> 7

tcgggctcac ccagtgacaa    20

<210> 8

<211> 20

<212> DNA

<213> Artificial Sequence

<220>

<223> Designed oligonucleotide primer for amplifying DNA fragment of

corresponding gene

<400> 8

atgggcattg cgtttgcagc    20

<210> 9

<211> 20

<212> DNA

<213> Artificial Sequence

<220>

<223> Designed oligonucleotide primer for amplifying DNA fragment of

corresponding gene

<400> 9

atgcagtgct ggcagcagga    20

<210> 10

<211> 20

<212> DNA

<213> Artificial Sequence

<220>

<223> Designed oligonucleotide primer for amplifying DNA fragment of

corresponding gene

<400> 10

tgctacaacc cacacgcaaa    20

<210> 11

<211> 20

<212> DNA

<213> Artificial Sequence

<220>
<223> Designed oligonucleotide primer for amplifying DNA fragment of
corresponding gene

<400> 11
tggcacccag cgactcgcca    20

<210> 12
<211> 20
<212> DNA
<213> Artificial Sequence

<220>
<223> Designed oligonucleotide primer for amplifying DNA fragment of
corresponding gene

<400> 12
ccgaagttcc aggtaacact    20

<210> 13
<211> 20
<212> DNA
<213> Artificial Sequence

<220>

<223> Designed oligonucleotide primer for amplifying DNA fragment of
corresponding gene

<400> 13
aggtaaggcc tttgagccca    20

<210> 14
<211> 20
<212> DNA
<213> Artificial Sequence

<220>
<223> Designed oligonucleotide primer for amplifying DNA fragment of
corresponding gene

<400> 14
actgttgtag tttagaaggc    20

<210> 15
<211> 20
<212> DNA
<213> Artificial Sequence

<220>
<223> Designed oligonucleotide primer for amplifying DNA fragment of
corresponding gene

<400> 15

gaaacaaata agaccataaa    20

<210> 16

<211> 20

<212> DNA

<213> Artificial Sequence

<220>

<223> Designed oligonucleotide primer for amplifying DNA fragment of

corresponding gene

<400> 16

cgccgagtga cagagacgct    20

<210> 17

<211> 20

<212> DNA

<213> Artificial Sequence

<220>

<223> Designed oligonucleotide primer for amplifying DNA fragment of

corresponding gene

<400> 17

tgggaacact gggtattctc    20

<210> 18

<211> 20

<212> DNA

<213> Artificial Sequence

<220>

<223> Designed oligonucleotide primer for amplifying DNA fragment of

corresponding gene

<400> 18

gtacatacag aagtatggct    20

<210> 19

<211> 20

<212> DNA

<213> Artificial Sequence

<220>

<223> Designed oligonucleotide primer for amplifying DNA fragment of

corresponding gene

<400> 19

tttgtgcatg aaacagagtt    20

<210> 20

<211> 20

<212> DNA
<213> Artificial Sequence

<220>
<223> Designed oligonucleotide primer for amplifying DNA fragment of
corresponding gene

<400> 20
gagctgctga cgcactgtga    20

<210> 21
<211> 20
<212> DNA
<213> Artificial Sequence

<220>
<223> Designed oligonucleotide primer for amplifying DNA fragment of
corresponding gene

<400> 21
gaaggcaaag cccactgctc    20

<210> 22
<211> 20
<212> DNA
<213> Artificial Sequence

<220>

<223> Designed oligonucleotide primer for amplifying DNA fragment of

corresponding gene

<400> 22

caaatttcat gagctattag    20

<210> 23

<211> 20

<212> DNA

<213> Artificial Sequence

<220>

<223> Designed oligonucleotide primer for amplifying DNA fragment of

corresponding gene

<400> 23

aagtcttggt cttttcaaaa    20

<210> 24

<211> 20

<212> DNA

<213> Artificial Sequence

<220>

<223> Designed oligonucleotide primer for amplifying DNA fragment of

corresponding gene

<400> 24
gcctttgcaa agcttttatt    20

<210> 25
<211> 20
<212> DNA
<213> Artificial Sequence

<220>
<223> Designed oligonucleotide primer for amplifying DNA fragment of
corresponding gene

<400> 25
aactcctcca gttctctcat    20

<210> 26
<211> 20
<212> DNA
<213> Artificial Sequence

<220>
<223> Designed oligonucleotide primer for amplifying DNA fragment of
corresponding gene

<400> 26
ggcaaacaac aatctcttgg    20

<210> 27

<211> 20

<212> DNA

<213> Artificial Sequence

<220>

<223> Designed oligonucleotide primer for amplifying DNA fragment of

corresponding gene

<400> 27

gtcgtgggtc tggaaagagt    20

<210> 28

<211> 20

<212> DNA

<213> Artificial Sequence

<220>

<223> Designed oligonucleotide primer for amplifying DNA fragment of

corresponding gene

<400> 28

cagccctctg agtctggaca    20

<210> 29

<211> 20

<212> DNA

<213> Artificial Sequence

<220>

<223> Designed oligonucleotide primer for amplifying DNA fragment of

corresponding gene

<400> 29

ccgcacccca atcctcttga    20

<210> 30

<211> 20

<212> DNA

<213> Artificial Sequence

<220>

<223> Designed oligonucleotide primer for amplifying DNA fragment of

corresponding gene

<400> 30

ggtcatgaca tttcctgtac    20

<210> 31

<211> 20

<212> DNA

<213> Artificial Sequence

<220>
<223> Designed oligonucleotide primer for amplifying DNA fragment of
corresponding gene

<400> 31
tgaagcaata gttggtgtcc    20

<210> 32
<211> 20
<212> DNA
<213> Artificial Sequence

<220>
<223> Designed oligonucleotide primer for amplifying DNA fragment of
corresponding gene

<400> 32
tgcgaggatt ttcaggacac    20

<210> 33
<211> 20
<212> DNA
<213> Artificial Sequence

<220>
<223> Designed oligonucleotide primer for amplifying DNA fragment of

corresponding gene

<400> 33
accctactat taaggcactt    20

<210> 34
<211> 20
<212> DNA
<213> Artificial Sequence

<220>
<223> Designed oligonucleotide primer for amplifying DNA fragment of
corresponding gene

<400> 34
acaatctcac tcatgacaga    20

<210> 35
<211> 20
<212> DNA
<213> Artificial Sequence

<220>
<223> Designed oligonucleotide primer for amplifying DNA fragment of
corresponding gene

<400> 35

gactctttca acttgagcag    20

<210> 36
<211> 20
<212> DNA
<213> Artificial Sequence

<220>
<223> Designed oligonucleotide primer for amplifying DNA fragment of
corresponding gene

<400> 36
atgagcttct tgccgttgtt    20

<210> 37
<211> 20
<212> DNA
<213> Artificial Sequence

<220>
<223> Designed oligonucleotide primer for amplifying DNA fragment of
corresponding gene

<400> 37
gtaaggccta ggcacagagg    20

<210> 38

<211> 20

<212> DNA

<213> Artificial Sequence

<220>

<223> Designed oligonucleotide primer for amplifying DNA fragment of

corresponding gene

<400> 38

taaaagatgc agtttgagag    20

<210> 39

<211> 20

<212> DNA

<213> Artificial Sequence

<220>

<223> Designed oligonucleotide primer for amplifying DNA fragment of

corresponding gene

<400> 39

gaaacatctg agcactatga    20

<210> 40

<211> 20

<212> DNA

<213> Artificial Sequence

<220>
<223> Designed oligonucleotide primer for amplifying DNA fragment of
corresponding gene

<400> 40
tgccattgtg cctcagcaca   20

## Description

### Technical Field

The present invention relates to a method for preparing a substrate for immobilizing ligands which is useful for preparation of a substrate having immobilized ligands such as a DNA chip, a saccharide chip or a protein chip.

### Background Art

With the progress in techniques for analyzing genomes, structures of genomes of various organisms have been (are being) elucidated. In parallel, development of techniques for analyzing functions of genomes or encoded functional molecules (e.g., proteins) has been promoted. Among these, a DNA chip (DNA microarray), a saccharide chip and a protein chip are noticeable techniques. A DNA chip is a microarray in which a number of different genes or fragments thereof (DNA fragments) are arrayed and immobilized onto a surface of a solid phase substrate such as a slide glass. It is very useful as a technique that remarkably promotes analyses of expression, mutation, polymorphism and the like of a gene. A saccharide chip is a microarray in which monosaccharides or oligosaccharides are arrayed and immobilized onto a surface of a solid phase substrate such as a slide glass. It is very useful as a technique that remarkably promotes analyses of saccharides, saccharide targets, interactions between them and the like. A protein chip is a microarray in which proteins, antibodies or fragments thereof are arrayed and immobilized. It is very useful as a technique that remarkably promotes analyses of proteins, antibodies, interactions between them and the like.

Immobilization of DNAs onto a substrate is a fundamental technique for preparing a DNA chip. Known methods are generally divided into two groups. One is a group of methods in which DNAs are chemically synthesized at the tips of linkers covalently attached to a substrate. Such methods are disclosed, for example, in Science, 251:767-773 (1991) and Nucleic Acids Research, 20:1679-1684 (1992). DNAs that can be immobilized by such methods are restricted to oligonucleotides, and the methods require special equipment for controlling the reactions. Thus, it cannot be said that they are general methods.

The other is a group of methods in which DNAs that have been synthesized beforehand or DNAs prepared by polymerase chain reactions (PCRs) (PCR amplification products) are covalently or noncovalently attached to a substrate. Such methods are disclosed, for example, in Science, 270:467-470 (1995) and Nucleic Acids Research, 22:5456-5465 (1994).

In an exemplary known immobilization method by noncovalent binding, a DNA dissolved in a salt solution such as SSC (sodium chloride/sodium citrate) is spotted, as it is or after denaturation, onto a slide glass (substrate) coated with a basic polycation (e.g., polylysine or polyethyleneimine), a basic silane-coupling agent having an amino group or the like, and the slide glass is then subjected to UV irradiation for immobilization. It is supposed that one can immobilize any DNAs according to such a method.

However, it is difficult to immobilize an oligonucleotide or a short DNA (0.3 kb or less) using this method. Furthermore, if a long DNA is to be immobilized onto a substrate via a noncovalent bond, the immobilized DNA tends to be detached from the substrate during a step of washing or hybridization with a target nucleic acid. This phenomenon is a factor that reduces the sensitivity of detection of a target nucleic acid. In addition, the method has a drawback that the background tends to be high due to nonspecific binding between a basic functional group (cation) remaining on a surface of a substrate and a target nucleic acid.

On the other hand, any DNAs including oligonucleotides can be immobilized according to a method of immobilization by covalent binding. However, since a substrate having a cationic group (e.g., an amino group) as a functional group is generally used, this method has a drawback that the background tends to be high like the above-mentioned one, resulting in reduction in detection sensitivity. A method in which the remaining amino groups are blocked by acetylation or the like is known, although the effect is insufficient.

A method in which a substrate having an anion group (e.g., a carboxyl group) as a functional group on its surface is used to reduce background is known (WO 01/02538). A substrate for immobilizing nucleic acids prepared according to this method can be used to detect a nucleic acid of interest with high sensitivity since background can be reduced as described above and because a number of DNA molecules can be covalently immobilized due to the surface treatment with a polyanion. According to this method, DNAs are immobilized onto a substrate via covalent bonds by surface treatment of the substrate with a polyanion (e.g., polyacrylic acid), generation of an active ester derivative of the polyanion on the surface, and spotting of DNAs.

Many steps are required for immobilizing DNAs onto a substrate via covalent bonds according to the above-mentioned method. Thus, a lot of labor and large amounts of reagents are required for using this method to prepare a substrate for immobilizing nucleic acids that enables detection of a nucleic acid of interest with high sensitivity. Accordingly, it has been disadvantageous in view of costs to actually prepare such a substrate and supply it on the market.

A method for preparing a DNA chip is described in WO 01/02538. According to this method, polyacrylic acid is fixed to an aminopropylsilanized slide glass by dehydration condensation using 1-[3-(dimethylamino)propyl]-3-ethylcarbodiimide hydrochloride. After washing, a dehydration condensation reaction is carried out using 1-[3-(-dimethylamino)propyl]-3-ethylcarbodiimide hydrochloride to form an N-hydroxysuccinimide ester. Thus, two steps of solid phase condensation reactions are conducted. An expensive reagent 1-[3-(-dimethylamino)propyl]-3-ethylcarbodiimide hydrochloride is required for the reactions. In addition, a lot of operations are required because washings with organic solvents are frequently conducted. Furthermore, the efficiencies of the condensation reactions all of which are conducted in a solid phase are inferior to those of liquid phase reactions. There has been a further problem that use of 1-[3-(-dimethylamino)propyl]-3-ethylcarbodiimide hydrochloride results in a low efficiency of the condensation reaction because it is weakly acidic in liquid.

For immobilization of a saccharide onto a substrate, it is possible to effectively utilize a glycosylamine which is also a very important intermediate for the preparation of a derivative of a monosaccharide or an oligosaccharide. An amino group specifically introduced to carbon at 1-position of a residue at its reducing end is very reactive, and can be coupled with an active ester of carboxylic acid or a compound having an isothiocyanate group or the like under mild conditions. By appropriately selecting a compound to be coupled with a glycosylamine, it is possible to introduce a monosaccharide or an oligosaccharide into a synthetic polymer (e.g., polyacrylamide), to immobilize a monosaccharide or an oligosaccharide onto a solid phase support, to fluorescently label a monosaccharide or an oligosaccharide, or to confer a hydrophobic property on a monosaccharide or an oligosaccharide that is originally hydrophilic (Neoglycoconjugate, pp. 199-223, Y.C. Lee and Reiko C. Lee (eds.), Academic Press, 1994).

One feature of a glycosylamine is the fact that it can be conveniently prepared. Preparation of a glycosylamine does not require reactions of blocking/deblocking of hydroxyl groups which are frequently utilized for general saccharide synthesis reactions. It can be prepared only by dissolving a monosaccharide or an oligosaccharide in a saturated ammonium bicarbonate aqueous solution, and allowing the resulting solution to stand for 3 to 6 days (Journal of Carbohydrate Chemistry, 8:597-611 (1989)).

A protein can be immobilized onto a substrate via a functional group of an N-terminal, C-terminal or internal amino acid residue of the protein, or a sugar chain attached to the protein (Biophys. J., 70:2437-2441 (1996)). Alternatively, a protein may be immobilized as a fusion protein with a linker containing a residue highly reactive with a functional group on a surface of a substrate (also called an affinity tag) (WO 00/04389).

Biological molecules (e.g., lipids) or other compounds (e.g., drugs) other than the above-mentioned ones can be immobilized onto a substrate and then used. Furthermore, it is known that a cell (a microorganism, an animal cell, a plant cell) can be immobilized onto a substrate and used for various objects.

As described above, there are various ligands desired to be bound to a substrate. A high-performance substrate for immobilizing ligands is necessary and indispensable for a material having such ligands being immobilized thereon (e.g., a chip) to exert its ability sufficiently.

### Objects of Invention

The main object of the present invention is to provide a substrate for immobilizing ligands which can be used to prepare a chip that has an ability of detecting a ligand with high sensitivity equivalent to one prepared using a substrate for immobilizing ligands prepared according to the above-mentioned method and which is so practical in view of costs that it can be actually produced and supplied on the market, as well as a method for preparing the substrate and a material having immobilized ligands obtained using the substrate.

### Summary of Invention

The present inventors have intensively searched for a preparation method that is so practical in view of costs that it can be used to actually prepare and supply on the market a substrate for immobilizing ligands that can be used to detect a receptor of interest with high sensitivity. As a result, the present inventors have found a method for preparing a substrate for immobilizing ligands. According to the method, a substrate of which the surface has been aminated beforehand is directly treated with a polyacrylate ester reaction mixture. The number of steps and the amounts of reagents required for the method are much less than those required for a conventional method. The method can be used to prepare a chip that has an ability of detecting a ligand with high sensitivity equivalent to that of a substrate for immobilizing ligands prepared according to a conventional method. The method is so practical in view of costs that it can be used to actually prepare and supply on the market the substrate. Thus, the present invention has been completed.

The present invention is outlined as follows. The first aspect of the present invention relates to a substrate for immobilizing ligands coated with an activated polyanion which has been activated beforehand.

According to the first aspect, it is preferable that the activated polyanion is an activated carboxylate having a functional group susceptible to substitution or elimination. Polyacrylate succinimide ester can be preferably used as the activated carboxylate.

The second aspect of the present invention relates to a method for preparing a substrate for immobilizing ligands, the method comprising activating a polyanion into an activated polyanion on a polyanion-coated substrate for immobilizing ligands.

According to the second aspect, it is preferable that the activated polyanion is an activated carboxylate having a functional group susceptible to substitution or elimination. Polyacrylate succinimide ester can be preferably used as the activated carboxylate.

The third aspect of the present invention relates to a material having immobilized ligands in which nucleic acids are immobilized in predetermined regions on a surface of the substrate for immobilizing ligands of the first aspect.

### Detailed Description of the Invention

There is no specific limitation concerning a ligand according to the present invention as long as it is a molecule recognized and bound by a specific receptor. For example, the ligand is a nucleic acid, a sugar chain, a lipid, a peptide or a protein. The nucleic acid may be a short nucleic acid, a long nucleic acid, a single-stranded nucleic acid, a double-stranded nucleic acid, a DNA or an RNA. The DNA may be a double-stranded DNA, a single-stranded DNA or a cDNA. The RNA may be an mRNA. A short nucleic acid is defined to be a nucleic acid consisting of 2 to 100 nucleotides, and a long nucleic acid is defined to be a nucleic acid consisting of more than 100 nucleotides.

Examples of the ligands include agonists and antagonists of cell membrane receptors, toxins and venoms, epitopes of viruses, hormones (e.g., sedatives, opiates and steroids), hormone receptors, peptides, enzymes, substrates for enzymes, cofactors, drugs, lectins, saccharides, oligonucleotides, polynucleotides, nucleic acids, oligosaccharides, proteins, antigens, monoclonal antibodies, lectins, cells, tumor cells, bacteria, viruses, chemical substances and avidin.

There is no specific limitation concerning a receptor according to the present invention as long as it is capable of binding to the corresponding ligand. It may be a naturally occurring molecule or an artificial molecule. Examples of the receptors include nucleic acids, sugar chains, proteins, peptides, enzymes, cell surface proteins (receptors), glycoproteins and antibodies.

The properties of the contacting surfaces of a ligand and a receptor capable of binding to the ligand according to the present invention are complementary to each other. For example, the receptor may be a nucleic acid, a saccharide, a peptide or a protein that is capable of binding to a nucleic acid, a saccharide, a peptide or a protein. A labeled receptor may be used as the receptor.

There is no specific limitation concerning a polyanion according to the present invention as long as it is a compound that has two or more acidic functional groups and it can be used to attach a nucleic acid.

Hereinafter, the present invention will be described in detail.

### (1) The substrate for immobilizing ligands of the present invention

There is no specific limitation concerning a substrate used to immobilize ligands according to the present invention as long as it can be used as a substrate for a DNA chip, a protein chip, a saccharide chip, a biosensor or the like. For example, a non-porous material that has a smooth surface and that is not fluorescent (e.g., glass such as a fluorescence-free slide glass can be preferably used.

It is preferable that the substrate is subjected to surface treatment to retain a compound having an anionic functional group beforehand. A substrate treated with a silane-coupling agent or the like may be used if the treatment does not result in any inconvenience for the surface treatment.

A polymer having an acidic functional group such as a carboxyl group, a phosphate group or a sulfate group which is effective in preventing nonspecific binding of nucleic acids can be preferably used as a polyanion for the surface treatment of the substrate. Examples of such polymers which can be preferably used include, but are not limited to, polyacrylic acid, polyglutamic acid, polyaspertic acid and polyphosphoric acid.

The substrate for immobilizing nucleic acids of the present invention can be subjected to surface treatment after the acidic functional group of the polymer is activated. A method of attachment of a functional group susceptible to substitution or elimination can be preferably used for activating the functional group. Although there is no specific limitation concerning a leaving group, one described as a good leaving group in Cram & Hammond, Organic Chemistry, 4th edition, 1981, McGraw-Hill International Book Company may be preferably used. For example, a halogen group (e.g., an I, Br or Cl group), or an ester of an alkoxy group (e.g., a methoxy or ethoxy group) or an imide group (e.g., succinimide) can be preferably used. Alternatively, a phenolic substance (e.g., p-nitrophenol or pentafluorophenol) or a hydroxyamine substance (e.g., N-hydroxybenzotriazole or 3,4-dihydro-3-hydroxy-4-oxo-1,2,3-benzotriazine) may be used for activation.

### (2) The method for preparing a substrate for immobilizing ligands of the present invention

One embodiment of the method for preparing a substrate or immobilizing ligands of the present invention is shown below. A conventional method for preparing a substrate for immobilizing ligands comprises the following 12 steps: (1) amination of a slide glass → (2) coating with polyacrylic acid → (3) washing in dimethylformamide (DMF) → (4) washing in methanol → (5) washing in 0.3 N NaOH → (6) washing in distilled water (D.W.) → (7) washing in acetone → (8) drying (5 hours) → (9) esterification reaction → (10) washing in DMF → (11) washing in dichloromethane → (12) drying. One embodiment of the method for preparing a substrate for immobilizing ligands of the present invention comprises the following 6 steps: (1) amination of a slide glass → (2) synthesis of polyacrylate ester → (3) coating with a solution of polyacrylate ester → (4) washing in DMF → (5) washing in dichloromethane → (6) drying. The conventional preparation method comprises 12 steps. On the other hand, the preparation method of the present invention which comprises 6 steps can be used to prepare a substrate for immobilizing ligands that has an ability of detecting a ligand with high sensitivity. Thus, a chip that has an ability of detecting a ligand with high sensitivity of interest can be prepared according to the method of which the number of steps is a half of that of the conventional method.

According to the method for preparing a substrate for immobilizing ligands of the present invention, it is preferable to use polyacrylic acid as a polyanion, and a non-porous substrate (in particular, glass) as a substrate.

There is no specific limitation concerning a method for preparing a DNA to be immobilized onto the substrate for immobilizing ligands of the present invention. For example, a nucleic acid chemically synthesized using a DNA synthesizer or a nucleic acid enzymatically synthesized according to the PCR method, the ICAN method (WO 00/56877) or the like can be preferably used. Although it is not intended to limit the present invention, for example, the chemical synthesis method using a DNA synthesizer may be preferably used in case of a short nucleic acid, and the enzymatic method may be preferably used in case of a long nucleic acid (DNA).

One embodiment of the method for preparing a substrate for immobilizing ligands of the present invention is described below. A substrate for immobilizing ligands of interest can be obtained using the following six steps.
Step 1: For surface treatment of a slide glass, for example, an aminopropyltriethoxysilane coupling agent or the like is used to aminopropylsilanize the surface of the substrate.
Step 2: N-hydroxysuccinimide, 1-ethyl-3-(3-dimetylaminopropyl)-carbodiimide hydrochloride (hereinafter referred to as EDC) and 4-dimethylaminopyridine are added to polyacrylic acid dissolved in dimethylformamide. After dissolution, the pH is adjusted to 7 to 8 using triethylamine. The mixture is stirred to convert polyacrylic acid into an active ester.
Step 3: The reaction mixture prepared in step 2 or a dilution of the reaction mixture with dimethylformamide is used to treat the surface of the aminopropylsilanized slide glass.
Step 4: The slide glass prepared in step 3 is washed in dimethylformamide.
Step 5: The slide glass is further washed in dichloromethane.
Step 6: The washed slide glass is dried.

According to the preparation method of the present invention, an aminopropylsilanized slide glass is coated using a reaction of substituting amide of polyacrylate succinimide ester. Thus, the amount of the expensive reagent EDC to be used can be decreased. Furthermore, the amounts of solvents used for washing and the like can be decreased because the number of preparation steps is reduced by half. Thus, the method for preparing a substrate for immobilizing ligands is very useful in view of the total cost of preparation method, the cost of reagents, and reduction in the burden on environment.

Generation of polyacrylate succinimide ester by conversion of polyacrylic acid into an active ester in step 2 of the preparation method of the present invention can be verified as follows.

The reaction mixture obtained in step 2 is added dropwise to ice-cold water. The resulting precipitate is collected by filtration to obtain a white powder. The infrared absorption spectrum of the white powder is then examined for verification. Specifically, an unreacted carboxyl group results in carboxylate-specific absorption at 1,610-1,550 cm⁻¹ and 1,400 cm⁻¹. On the other hand, polyacrylate succinimide ester as the product results in ester carbonyl-specific absorption at 1,736 cm⁻¹, 1,205 cm⁻¹ and 1,069 cm⁻¹ instead of the above-mentioned absorption. Thus, conversion of polyacrylic acid into an active ester can be verified.

Furthermore, generation of polyacrylate succinimide ester of interest can be verified by subjecting polyacrylic acid and N-hydroxysuccinimide which are not subjected to reaction as well as the white powder as a reaction product obtained after reaction to development on thin-layer chromatography (TLC) and examining absorption upon exposure to ultraviolet ray at 254 nm. Specifically, the mobility (Rf value) of polyacrylic acid on thin-layer chromatography is unchanged even if it is converted into a succinimide ester because of the high molecular weight. However, since attachment of succinimide increases ultraviolet absorption, unreacted polyacrylic acid can be distinguished from the reaction product. Thus, generation of polyacrylate succinimide ester can be verified.

Polyacrylate succinimide ester used according to the preparation method of the present invention can be obtained as a white powder by reacting polyacrylic acid with N-hydroxysuccinimide, adding the reaction mixture dropwise to ice-cold water and filtering the resulting precipitate. Polyacrylate succinimide ester as the white powder is then dissolved in dimethylformamide, and the solution is used to treat a surface of an aminopropylsilanized slide glass. Then, a substrate for immobilizing ligands which enables preparation of a chip that has an ability of detecting a ligand with high sensitivity of interest can be prepared. Thus, polyacrylate succinimide ester can be stored in a form of powder stably for a long period of time.

### (3) Immobilization of ligands

The following method can be used for immobilizing DNAs, saccharides or proteins onto a substrate for immobilizing ligands prepared according to the method of the present invention.

The method as described in WO 01/02538 can be applied if a DNA is to be immobilized. Specifically, a derivative of a DNA to be immobilized that is modified at its 5' end with an amino group, a thiol group, a phosphate group, an aldehyde group or the like, or a derivative having a crosslinking agent or a linker as a spacer (e.g., alkylamine) being attached at the modified 5' end is prepared. The modification at the 5' end is particularly useful for immobilization of a short nucleic acid such as an oligonucleotide. Furthermore, immobilization may be carried out by internally introducing an appropriate functional group into a DNA strand. DNA synthesis may be conducted in the presence of an appropriate modified nucleotide in this case. Alternatively, a DNA may be chemically modified. For example, the Label It reagent (Mirus) may be used for modifying a nucleic acid. A nucleic acid of interest can be immobilized onto a substrate as follows: the modified nucleic acid is dissolved in a solution suitable for immobilization (e.g., 20 mM 3-morpholinopropanesulfonic acid (MOPS) buffer (pH 7.5) or 50 mM carbonate buffer (pH 9.5)) at a concentration of 0.01-2.0 mg/ml, preferably 0.1-1.0 mg/ml; and the solution is contacted, as it is or after denaturation, with the substrate of the present invention in which a carboxylic acid has been converted into an activated ester by surface treatment. Specifically, a given amount of the DNA solution is spotted on a substrate having the activated ester on the surface using an instrument for preparing DNA chips (a DNA arrayer). After incubating it in a humidified incubator for 30-60 minutes, the substrate is then washed in 2% SDS (sodium dodecyl sulfate) followed by distilled water. Furthermore, the substrate having immobilized nucleic acids may optionally be immersed in 0.3 N NaOH at room temperature for 5 minutes or in boiling water for 2 minutes to denature nucleic acids, washed in distilled water followed by absolute ethanol, and dried. By this treatment, activated esters that have not reacted with a nucleic acid are hydrolyzed into carboxylate groups (i.e., negatively charged). Since the acidic groups prevent nonspecific electrostatic binding between a probe nucleic acid and the substrate, a procedure of blocking with succinic anhydride which is generally required for a polylysine slide glass can be omitted. Furthermore, the acidic groups reduce background signals.

In addition to DNAs, various ligands can be immobilized onto the substrate for immobilizing ligands of the present invention. The most efficient method for the ligand may be selected. Immobilization may be carried out using a method suitable for the properties of the ligand and the activated solid phase substrate. There is no specific limitation concerning a solvent that can be used for dissolving or suspending a ligand upon ligand immobilization. For example, an aqueous solution, dimethyl sulfoxide, dimethylformamide or a mixture thereof is preferable. The ligand solution or suspension may contain a detergent. For example, addition of glycerol, polyethylene glycol, a saccharide or a salt may be effective for adjusting the shape of spots or for preventing spots from drying.

A saccharide having an amino group or a thiol group can be immobilized onto a solid phase substrate having a carboxylic acid activated, for example, using an N-hydroxysuccinimide ester group or a halogen group. For example, a glycosylamine which has a highly reactive amino group is preferable for immobilization onto the substrate of the present invention.

In addition, it is possible to form a covalent bond between a saccharide target and a solid phase substrate using a crosslinking agent and/or a reaction promoter.

Many crosslinking agents are commercially available. For example, compounds having various reactive groups or molecular lengths such as ethylene glycol-bis-(succinimidyl succinate) or N-(ε-maleimidocaproyloxy)succinimide ester are commercially available from Pierce. A crosslinking agent can be appropriately selected according to the present invention from such crosslinking agents depending on the combination of the solid phase substrate and the saccharide target.

Although it is not intended to limit the present invention, for example, 1-ethyl-3-(3-dimethylaminopropyl)carbodiimide, dicyclohexylcarbodiimide or a salt thereof can be preferably used as a reaction promoter.

There is no specific limitation concerning the sequence of contacts among three or four substances (i.e., a ligand, a solid phase substrate, and a crosslinking agent and/or a reaction promoter) upon immobilization of a ligand onto a solid phase substrate. For example, the sequence of contacts may be as follows.
(1) The three or four substances are contacted to each other at the same time;
(2) The ligand and the solid phase substrate are contacted to each other, and the crosslinking agent and/or the reaction promoter is(are) then contacted thereto; or
(3) One of the ligand and the solid phase substrate is contacted to the crosslinking agent and/or the reaction promoter, and the remaining one is then contacted thereto.

If the ligand is a cell such as a Gram-positive bacterium, a Gram-negative bacterium, a fungus, a yeast, a protozoan, a helminth, a cultured animal cell or a cultured plant cell, the cell may be either living or dead. The above-mentioned method can be used for immobilization onto a surface of a solid phase. For example, the cell can be immobilized onto the substrate for immobilizing ligands of the present invention utilizing amino groups on the surface of the cell.

A cell can be fixed using a commonly used microbiological or histochemical fixation method. Fixation means inactivation of a cell, and should be conceptually distinguished from immobilization onto a solid phase substrate or solid phase immobilization. Examples of microbiological or histochemical fixation methods include fixation by drying, fixation using 70% to 90% ethanol and fixation using glutaraldehyde or formaldehyde. The microbiological or histochemical fixation may be carried out prior to or following immobilization onto a solid phase substrate or solid phase immobilization.

A protein can be immobilized onto the substrate for immobilizing ligands of the present invention via an activated ester-reactive functional group of an N-terminal, C-terminal or internal amino acid residue of the protein, or a sugar chain attached to the protein. Alternatively, a protein to be immobilized can be immobilized as a fusion protein with a linker containing a residue highly reactive with an activated ester on a surface of the substrate (also called an affinity tag). Attachment via a linker is advantageous in that inactivation of a protein on a surface of a substrate can be suppressed. In particular, if an antibody or a fragment thereof is to be immobilized, immobilization as a fusion protein with a linker is preferable.

There is no specific limitation concerning a linker. Any hetero- or homopeptide consisting of 2 to 100 residues may be used. Examples of the homopeptides include polycysteine, polylysine, polyarginine and polyhistidine.

The linker may contain one or more nonnatural amino acid(s). A method in which suppressor tRNA that recognizes amber codon is used may be used for introducing a nonnatural amino acid (Science, 244:182-188 (1989); Methods Enzym., 202:301-336 (1991); Chem. Biol., 3:1033-1038 (1996)).

If biotin or an antigen is to be used as a linker, it is chemically crosslinked to a protein.

There is no specific limitation concerning a signal-emitting substance used for detecting a receptor bound to a ligand according to the present invention. A fluorescent substance or a luminescent substance may be used. For example, a chemiluminescent substance such as AMPPD or a fluorescent substance such as fluorescein, Cascade Blue, Oregon Green, BODIPY, Rhodamine Green, Alexa Fluor, Texas Red, Cy3 or Cy5 can be preferably used.

As described above, the substrate for immobilizing ligands of the present invention has an ability of detecting a receptor with high sensitivity equivalent to that for a substrate prepared according to a conventional method (e.g., the method as described in WO 01/02538). Since the method for preparing a substrate for immobilizing ligands of the present invention comprises a less number of steps than a conventional method, the total cost of preparation method and the cost of reagents to be used can be reduced, and a high-quality and low-cost substrate for immobilizing ligands can be provided on the market. In addition, since the amounts of reagents to be used can be reduced, the burden on global environment can be reduced.

### Examples

The following Examples further illustrate the present invention in detail but are not to be construed to limit the scope thereof.

### Example 1: Preparation of polyacrylic acid converted into active ester

(1) 1.0 g of polyacrylic acid (molecular weight: 1,000,000; Wako Pure Chemical Industries) was dissolve in 200 ml of dimethylformamide. 4.8 g of N-hydroxysuccinimide (Nacalai Tesque), 8.0 g of N-ethyl-N'-dimethylaminopropylcarbodiimide hydrochloride (Peptide Institute) and 52 mg of 4-dimethylaminopyridine (Nacalai Tesque) were added thereto while cooling on ice. After the reagents were dissolved, the pH was adjusted to 7 to 8 using triethylamine. The resulting mixture was stirred overnight at room temperature. This solution is designated as a polyacrylate ester solution 1.
(2) The reaction mixture obtained in Example 1-(1) was added dropwise to three volumes of ice-cold distilled water. The resulting precipitate was collected by filtration, washed in water followed by ethanol, and dried under reduced pressure to obtain 1.7 g of polyacrylate succinimide ester as a white powder (yield: 72.6%).
(3) The infrared absorption spectrum of polyacrylate succinimide ester as the reaction product obtained in Example 1-(2) was measured. An unreacted carboxyl group results in carboxylate-specific absorption at 1,610-1,550 cm⁻¹ and 1,400 cm⁻¹. The white powder resulted in ester carbonyl-specific absorption at 1,735.8 cm⁻¹, 1,205.4 cm⁻¹ and 1,068.5 cm⁻¹ instead of the above-mentioned absorption. Furthermore, polyacrylate succinimide ester was analyzed using thin-layer chromatography (TLC). Polyacrylic acid, N-hydroxysuccinimide and the white powder which had been dissolved in dimethylformamide were spotted onto a silica gel thin-layer plate (MERCK TLC aluminiumsheets Silicagel 60F254, Merck). After air-drying, development was carried out using chloroform/methanol/acetic acid (9:1:1, v/v).

The mobility (Rf value) of polyacrylic acid on thin-layer chromatography is unchanged even if it is converted into a succinimide ester because of the high molecular weight. However, since attachment of succinimide increases ultraviolet absorption, unreacted polyacrylic acid can be distinguished from the reaction product. After development, the thin-layer plate was air-dried and exposed to ultraviolet ray at 254 nm. Then, intense absorption was observed around the center for N-hydroxysuccinimide. Intense absorption was observed at the origin (Rf value = 0) for the white powder. No absorption was observed for polyacrylic acid. Based on these results, generation of polyacrylate succinimide ester was verified.

### Example 2: Surface treatment of slide glass with polyacrylic acid converted into active ester

(1) A microscopic slide glass (Matsunami Glass) was immersed sequentially in 2 M nitric acid, 2 M sodium hydroxide solution and distilled water and sonicated each for 10 minutes. 3-Aminopropyltriethoxysilane (Nacalai Tesque) was added to 95% ethanol solution at a final concentration of 8%. The treated slide glass was immersed in this solution for 2 minutes, and then treated in an oven at 100°C for 10 minutes to aminopropylsilanize the surface of the slide glass. The aminopropylsilanized slide glass was immersed overnight in the polyacrylate ester solution 1, or a solution of the white powder of polyacrylate succinimide ester obtained in Example 1 in 99.5% dimethylformamide (hereinafter referred to as a polyacrylate ester solution 2). The slide glass was washed sequentially by sonication in dimethylformamide and methylene chloride, and then dried under reduced pressure.
(2) A human model DNA chip was prepared using the substrate for immobilizing nucleic acids prepared in Example 2-(1), and assessed as follows. Twenty human genes listed in Tables 1 and 2 were used as test nucleic acids. The nucleic acid fragments were prepared using PCRs. Nucleotide sequences of primers used for PCR are shown in SEQ ID NOS:1 to 40. The primer pairs used for PCRs for the respective genes are shown in Tables 1 and 2. The 5' primers were modified at their 5' ends with amino groups.

**Table 1**

| | Gene name | GenBank accession no. | SEQ ID NO | |
|---|---|---|---|---|
| | | | 5' | 3' |
| 1 | Zona pellucida glycoprotein 3A (sperm receptor) | NM_007155 | 1 | 21 |
| 2 | Wingless-type MMTV integration site family, member 5A | NM_003392 | 2 | 22 |
| 3 | Wingless-type MMTV integration site family member 2 | NM_003391 | 3 | 23 |
| 4 | Villin 2 (ezrin) | NM_003379 | 4 | 24 |
| 5 | Vascular cell adhesion molecule 1 | NM_001078 | 5 | 25 |
| 6 | v-kit Hardy-Zuckerman 4 feline sarcoma viral oncogene homolog | NM_000222 | 6 | 26 |
| 7 | v-akt murine thymoma viral oncogene homolog 1 | NM_005163 | 7 | 27 |
| 8 | Transforming growth factor, beta receptor III (betaglycan, 300kD) | NM_003243 | 8 | 28 |
| 9 | EphA2 | NM_004431 | 9 | 29 |
| 10 | Tumor necrosis factor, alpha-induced protein 6 | NM_007115 | 10 | 30 |

**Table 2**

| | Gene name | GenBank accession no. | SEQ ID NO | |
|---|---|---|---|---|
| | | | 5' | 3' |
| 11 | Transforming growth factor, beta 1 | NM_000660 | 11 | 31 |
| 12 | Interferon-stimulated transcription factor 3, gamma (48kD) | NM_006084 | 12 | 32 |
| 13 | v-rel avian reticuloendotheliosis viral oncogene homolog A (nuclear factor of kappa light polypeptide gene enhancer in B-cells 3 (p65)) | NM_021975 | 13 | 33 |
| 14 | Transcription elongation factor A (SII), 1 | NM_006756 | 14 | 34 |
| 15 | Selectin L (lymphocyte adhesion molecule 1) | NM_000655 | 15 | 35 |
| 16 | ribosomal protein S5 | NM_001009 | 16 | 36 |
| 17 | ras homolog gene family, member G (rho G) | NM_001665 | 17 | 37 |
| 18 | retinoic acid receptor, beta | NM_000965 | 18 | 38 |
| 19 | RAD52 (S. cerevisiae) homolog | NM_002879 | 19 | 39 |
| 20 | tyrosine kinase 2 | NM_003331 | 20 | 40 |

After PCR, the 20 amplified fragments were purified according to a conventional method, and dissolved in TaKaRa Solution T (Takara Bio) at concentrations of 0.3 µg/µl. The thus obtained solutions were used for spotting. The solutions were spotted onto the activated polyacrylate ester-coated slide glass prepared in Example 2-(1) using Affymetrix 417 Arrayer (Affymetrix). The slide glass was washed in 0.2% SDS and twice in distilled water and treated with 0.3 N sodium hydroxide solution at room temperature for 5 minutes. Then, the slide glass was washed in distilled water for 5 minutes, heated at 95°C for 2 minutes, rinsed with ice-cold 100% ethanol, and dried by centrifugation. The thus obtained substrate having immobilized nucleic acids was used as a DNA chip in the subsequent reaction.
(3) Confirmation by hybridization was carried out as follows. Briefly, salmon sperm DNA was fluorescently labeled with Cy3 using Label IT Cy3 Label Kit (Mirus) according to the instructions attached to the kit, and then purified. The fluorescently labeled salmon sperm DNA fragment was diluted with a hybridization solution to a final concentration of 0.1 µg/µl. Denaturation was carried out using distilled water, and Label IT buffers D1 and N1. Then, the DNA was dissolved in a hybridization buffer at a final concentration of 0.01 µg/µl.

About 9 µl of the hybridization solution was dripped onto a cover glass. The cover glass was covered with the slide glass with the DNA-immobilized side down so carefully that no bubble was included. The covered slide glass was turned upside down so carefully that the cover glass did not shift, placed in a humidified incubator, and incubated at 37°C for 30 minutes. After incubation, the cover glass was removed in 2 x SSC at room temperature. The slide glass was washed for 5 minutes in 0.2 x SSC containing 0.1% SDS followed by 0.2 x SSC, subjected to low-speed centrifugation at about 1,000 rpm to remove water, and air-dried.

After air-drying, the slide glass was subjected to measurement at an excitation wavelength of 532 nm and an emission wavelength of 570 nm using Affymetrix 418™ Array Scanner (Affymetrix). As a result, fluorescent signals were observed at all spots on the slide glass.

### Example 3

(1) A substrate for immobilizing nucleic acids prepared using the polyacrylate ester solution 1 according to the method as described in Example 2 and a substrate for immobilizing nucleic acids prepared according to the method as described in WO 01/02538 were compared to each other based on the detection sensitivities observed for human model DNA chips prepared using them. Specifically, spotting solutions for the 20 human genes as described in Example 2 prepared using the same test nucleic acids as those described in Example 2 were used. The solutions were spotted onto the slide glass for immobilizing nucleic acids prepared in Example 2 or a slide glass for immobilizing nucleic acids prepared according to the conventional method using Affymetrix 417 Arrayer. The slide glasses were washed in 0.2% SDS and twice in distilled water and treated with 0.3 N sodium hydroxide solution at room temperature for 5 minutes. Then, the slide glasses were washed in distilled water for 5 minutes, heated at 95°C for 2 minutes, rinsed with ice-cold 100% ethanol, and dried by centrifugation. The thus obtained substrates having immobilized nucleic acids were used as DNA chips in the subsequent reactions.
(2) Preparation of labeled cDNAs for detection and hybridization were carried out as follows. Briefly, polyA(+) RNAs were prepared from human HL-60 cells (Dainippon Pharmaceutical) and human A431 cells (Dainippon Pharmaceutical) using TRIzol Reagent (Gibco BRL) and Oligotex-dT30 <Super> (Takara Bio) according to the protocols for the kits. A Cy3-labeled cDNA and a Cy5-labeled cDNA were prepared using 1 µg of the polyA(+) RNA from HL-60 cells and 1 µg of the polyA(+) RNA from A431 cells as templates, respectively, as well as RNA Fluorescence Labeling Core Kit (M-MLV Version) (Takara Bio) according to the protocol for the kit, and then purified. Hybridization between the two DNA chips prepared in Example 3-(1) and a mixture of the Cy3-labeled cDNA and the Cy5-labeled cDNA, washing and drying were carried out according to the instructions attached to IntelliGene (Takara Bio). Fluorescence images for Cy3 (an excitation wavelength of 532 nm and an emission wavelength of 570 nm) and Cy5 (an excitation wavelength of 635 nm and an emission wavelength of 660 nm) and were obtained using Affymetrix 428 Array Scanner (Affymetrix). The signal intensities for the respective spots and the background signal intensity were quantified using an image quantification analysis software ImaGene 4.1 (Biodiscovery). Averages of values obtained by subtracting the background signal intensity from the signal intensities for the respective spots (hybridization signal intensities) and averages of ratios of the signal intensities for the respective spots to the background signal intensity (signal to noise ratios) were calculated for the 20 genes. As a result, the hybridization signal intensities and the signal to noise ratios observed for the DNA chip prepared using the slide glass having immobilized nucleic acids according to the preparation method of the present invention were equivalent to or greater than those observed for the DNA chip prepared using the slide glass having immobilized nucleic acids according to the conventional method. Based on these results, it was confirmed that, like the substrate for immobilizing nucleic acid prepared according to the conventional method, the substrate for immobilizing nucleic acids prepared according to the preparation method of the present invention can be preferably used as a substrate for immobilizing nucleic acids that enables preparation of a DNA chip that has an ability of detecting a nucleic acid with high sensitivity.

### Example 4

### (1) Conversion of polyacrylic acid into active ester

1.0 g of polyacrylic acid (molecular weight: 1,000,000) was dissolve in 200 ml of dimethylformamide. 4.8 g of N-hydroxysuccinimide, 8.0 g of N-ethyl-N'-dimethylaminopropyl-carbodiimide hydrochloride and 52 mg of 4-dimethylaminopyridine were added to the solution while cooling on ice. After the reagents were dissolved, the pH was adjusted to 7 to 8 using triethylamine. The resulting mixture was stirred overnight at room temperature.

### (2) Preparation of coating agent

The reaction mixture obtained in Example 4-(1) was filtered through a Buchner funnel equipped with a filter paper and covered with Celite 545 which had been washed with dimethylformamide. The volume of the filtrate was adjusted to 200 ml with dimethylformamide to obtain a coating agent.

### (3) Surface treatment of slide glass

A microscopic slide glass was subjected to 10-minutes sonications in 2 M nitric acid, 2 M sodium hydroxide and distilled water. 3-Aminopropyltriethoxysilane was added to 95% ethanol solution at a final concentration of 4%. The sonicated slide glass was immersed in this solution for 30 minutes, and then treated in an oven at 100°C for 10 minutes to aminopropylsilanize the surface of the slide glass.

The aminopropylsilanized slide glass was immersed for 1 hour in the coating agent obtained in Example 4-(2). The slide glass was washed sequentially by sonication in dimethylformamide and acetonitrile, and then dried under reduced pressure.

### (4) Determination of immobilization rates

A Cy3-labeled and end-aminated DNA fragment and a nonlabeled and end-aminated DNA fragment were prepared by PCRs using a Cy3-labeled primer having an amino group at its end and a nonlabeled primer having an amino group at its end which had been synthesized beforehand. Solutions of the Cy3-labeled and end-aminated DNA fragment and the nonlabeled and end-aminated DNA fragment were mixed with TaKaRa Solution I (Takara Bio).

The resulting mixture was spotted onto the slide glass prepared in Example 4-(3) using Affymetrix 417 Arrayer. Measurement was carried out at an excitation wavelength of 532 nm and an emission wavelength of 570 nm using Affymetrix 428 Array Scanner. As a result, fluorescent signals were observed at all spots on the slide glass.

After measurement, the slide glass was washed sequentially in 0.2% SDS, Milli-Q water, 0.3 N sodium hydroxide, Milli-Q water, Milli-Q water at 100°C and ice-cold ethanol, subjected to low-speed centrifugation at about 1,000 rpm to remove water, and air-dried. After air-drying, the slide glass was subjected to measurement at an excitation wavelength of 532 nm and an emission wavelength of 570 nm using Affymetrix 428 Array Scanner. The image obtained by the measurement was subjected to quantification using a microarray quantification software ImaGene 4.2, and DNA immobilization rates were calculated using a clustering software GeneSight V3.0.7.

As a result, the rates of DNA immobilization onto the slide glass of Example 4-(3) ranged from 13 to 26%, and the average value was about 20%. The rates of DNA immobilization onto the slide glass of Example 3 ranged from 23 to 34%, and the average value was about 28%. Equal or more DNA immobilization rates were observed even if the period of immersion in the coating agent was shortened from 16 hours to 1 hour.

### Example 5

### (1) Conversion of polyacrylic acid into active ester

3.25 g of polyacrylic acid (molecular weight: 1,000,000) was dissolve in 650 ml of dimethylformamide. 14.6 g of N-hydroxysuccinimide and 22.75 ml of N-ethyl-N'-dimethylaminopropyl-carbodiimide were added to the solution while cooling on ice. After the reagents were dissolved, the resulting mixture was stirred overnight at room temperature.

### (2) Preparation of coating agent

The reaction mixture obtained in Example 5-(1) was filtered through a Buchner funnel equipped with a filter paper and covered with Celite 545 which had been washed with dimethylformamide. The volume of the filtrate was adjusted to 1.3 1 with dimethylformamide to obtain a coating agent.

### (3) Surface treatment of slide glass

A microscopic slide glass was subjected to 10-minutes sonications in 2 M nitric acid, 2 M sodium hydroxide and distilled water. 3-Aminopropyltriethoxysilane was added to 95% ethanol solution at a final concentration of 4%. The sonicated slide glass was immersed in this solution for 30 minutes, and then treated in an oven at 100°C for 10 minutes to aminopropylsilanize the surface of the slide glass.

The aminopropylsilanized slide glass was immersed for 1 hour in the coating agent obtained in Example 5-(2). The slide glass was washed sequentially by sonication in dimethylformamide and acetonitrile, and then dried under reduced pressure.

### (4) Determination of immobilization rates

A Cy3-labeled and end-aminated DNA fragment and a nonlabeled and end-aminated DNA fragment were prepared by PCRs using a Cy3-labeled primer having an amino group at its end and a nonlabeled primer having an amino group at its end which had been synthesized beforehand. Solutions of the Cy3-labeled and end-aminated DNA fragment and the nonlabeled and end-aminated DNA fragment were mixed with TaKaRa Solution I (Takara Bio).

The resulting mixture was spotted onto the slide glass prepared in Example 5-(3) using Affymetrix 417 Arrayer. Measurement was carried out at an excitation wavelength of 532 nm and an emission wavelength of 570 nm using Affymetrix 428 Array Scanner. As a result, fluorescent signals were observed at all spots on the slide glass.

After measurement, the slide glass was washed sequentially in 0.2% SDS, Milli-Q water, 0.3 N sodium hydroxide, Milli-Q water, Milli-Q water at 100°C and ice-cold ethanol, subjected to low-speed centrifugation at about 1,000 rpm to remove water, and air-dried. After air-drying, the slide glass was subjected to measurement at an excitation wavelength of 532 nm and an emission wavelength of 570 nm using Affymetrix 428 Array Scanner. The image obtained by the measurement was subjected to quantification using a microarray quantification software ImaGene 4.2, and DNA immobilization rates were calculated using a clustering software GeneSight V3.0.7.

As a result, the rates of DNA immobilization onto the slide glass of Example 5-(3) ranged from 37 to 50%. Equal or more DNA immobilization rates were observed even if N-ethyl-N'-dimethylaminopropyl-carbodiimide hydrochloride was replaced by N-ethyl-N'-dimethylaminopropyl-carbodiimide.

### Example 6

### (1) Conversion of polyacrylic acid into active ester

Polyacrylate ester was prepared as described in Example 5-(1).

### (2) Preparation of coating agent

A coating agent was prepared as described in Example 5-(2).

### (3) Surface treatment of slide glass

A microscopic slide glass was subjected to 10-minutes sonications in 2 M nitric acid, 2 M sodium hydroxide and distilled water. 3-Aminopropyltriethoxysilane was added to 95% ethanol solution at a final concentration of 4%. The sonicated slide glass was immersed in this solution for 30 minutes, and then treated in an oven at 100°C for 10 minutes to aminopropylsilanize the surface of the slide glass.

The aminopropylsilanized slide glass was immersed for 1 hour in the coating agent obtained in Example 6-(2). The slide glass was washed sequentially in dimethylformamide and acetonitrile, and then dried under reduced pressure.

### (4) Repetitive use of coating agent - 1

A newly prepared aminopropylsilanized slide glass was immersed for 1 hour in the coating agent used in Example 6-(3). The slide glass was washed sequentially by sonication in dimethylformamide and acetonitrile, and then dried under reduced pressure.

### (5) Repetitive use of coating agent - 2

A newly prepared aminopropylsilanized slide glass was immersed for 1 hour in the coating agent used in Example 6-(4). The slide glass was washed sequentially by sonication in dimethylformamide and acetonitrile, and then dried under reduced pressure.

### (6) Repetitive use of coating agent - 3

A newly prepared aminopropylsilanized slide glass was immersed for 1 hour in the coating agent used in Example 6-(5). The slide glass was washed sequentially by sonication in dimethylformamide and acetonitrile, and then dried under reduced pressure.

### (7) Determination of immobilization rates

A Cy3-labeled and end-aminated DNA fragment and a nonlabeled and end-aminated DNA fragment were prepared by PCRs using a Cy3-labeled primer having an amino group at its end and a nonlabeled primer having an amino group at its end which had been synthesized beforehand. Solutions of the Cy3-labeled and end-aminated DNA fragment and the nonlabeled and end-aminated DNA fragment were mixed with TaKaRa Solution I (Takara Bio).

The resulting mixture was spotted onto the slide glasses prepared in Example 6-(3), (4), (5) and (6) using Affymetrix 417 Arrayer. Measurements were carried out at an excitation wavelength of 532 nm and an emission wavelength of 570 nm using Affymetrix 428 Array Scanner. As a result, fluorescent signals were observed at all spots on all the slide glasses.

After measurement, the slide glasses were washed sequentially in 0.2% SDS, Milli-Q water, 0.3 N sodium hydroxide, Milli-Q water, Milli-Q water at 100°C and ice-cold ethanol, subjected to low-speed centrifugation at about 1,000 rpm to remove water, and air-dried. After air-drying, the slide glasses were subjected to measurements at an excitation wavelength of 532 nm and an emission wavelength of 570 nm using Affymetrix 428 Array Scanner. The images obtained by the measurements were subjected to quantification using a microarray quantification software ImaGene 4.2, and DNA immobilization rates were calculated using a clustering software GeneSight V3.0.7.

As a result, the rates of DNA immobilization onto the slide glasses of Example 6-(3), Example 6-(4), Example 6-(5) and Example 6-(6) ranged from 41 to 45%, from 42 to 48%, from 39 to 42% and from 39 to 46%, respectively. The average values were about 40% or more in all cases. Thus, it was confirmed that the ability of immobilizing DNAs equivalent to the initial one was retained after the coating agent was repetitively used.

### Industrial Applicability

The present invention provides a low-cost substrate for immobilizing ligands that enables detection of a receptor of interest with high sensitivity. Furthermore, a material having immobilized ligands which can be used to detect and analyze a target receptor with high sensitivity is provided using the substrate.

### Sequence Listing Free Text

SEQ ID NO:1: Designed oligonucleotide primer for amplifying DNA fragment of corresponding gene
SEQ ID NO:2: Designed oligonucleotide primer for amplifying DNA fragment of corresponding gene
SEQ ID NO:3: Designed oligonucleotide primer for amplifying DNA fragment of corresponding gene
SEQ ID NO:4: Designed oligonucleotide primer for amplifying DNA fragment of corresponding gene
SEQ ID NO:5: Designed oligonucleotide primer for amplifying DNA fragment of corresponding gene
SEQ ID NO:6: Designed oligonucleotide primer for amplifying DNA fragment of corresponding gene
SEQ ID NO:7: Designed oligonucleotide primer for amplifying DNA fragment of corresponding gene
SEQ ID NO:8: Designed oligonucleotide primer for amplifying DNA fragment of corresponding gene
SEQ ID NO:9: Designed oligonucleotide primer for amplifying DNA fragment of corresponding gene
SEQ ID NO:10: Designed oligonucleotide primer for amplifying DNA fragment of corresponding gene
SEQ ID NO:11: Designed oligonucleotide primer for amplifying DNA fragment of corresponding gene
SEQ ID NO:12: Designed oligonucleotide primer for amplifying DNA fragment of corresponding gene
SEQ ID NO:13: Designed oligonucleotide primer for amplifying DNA fragment of corresponding gene
SEQ ID NO:14: Designed oligonucleotide primer for amplifying DNA fragment of corresponding gene
SEQ ID NO:15: Designed oligonucleotide primer for amplifying DNA fragment of corresponding gene
SEQ ID NO:16: Designed oligonucleotide primer for amplifying DNA fragment of corresponding gene
SEQ ID NO:17: Designed oligonucleotide primer for amplifying DNA fragment of corresponding gene
SEQ ID NO:18: Designed oligonucleotide primer for amplifying DNA fragment of corresponding gene
SEQ ID NO:19: Designed oligonucleotide primer for amplifying DNA fragment of corresponding gene
SEQ ID NO:20: Designed oligonucleotide primer for amplifying DNA fragment of corresponding gene
SEQ ID NO:21: Designed oligonucleotide primer for amplifying DNA fragment of corresponding gene
SEQ ID NO:22: Designed oligonucleotide primer for amplifying DNA fragment of corresponding gene
SEQ ID NO:23: Designed oligonucleotide primer for amplifying DNA fragment of corresponding gene
SEQ ID NO:24: Designed oligonucleotide primer for amplifying DNA fragment of corresponding gene
SEQ ID NO:25: Designed oligonucleotide primer for amplifying DNA fragment of corresponding gene
SEQ ID NO:26: Designed oligonucleotide primer for amplifying DNA fragment of corresponding gene
SEQ ID NO:27: Designed oligonucleotide primer for amplifying DNA fragment of corresponding gene
SEQ ID NO:28: Designed oligonucleotide primer for amplifying DNA fragment of corresponding gene
SEQ ID NO:29: Designed oligonucleotide primer for amplifying DNA fragment of corresponding gene
SEQ ID NO:30: Designed oligonucleotide primer for amplifying DNA fragment of corresponding gene
SEQ ID NO:31: Designed oligonucleotide primer for amplifying DNA fragment of corresponding gene
SEQ ID NO:32: Designed oligonucleotide primer for amplifying DNA fragment of corresponding gene
SEQ ID NO:33: Designed oligonucleotide primer for amplifying DNA fragment of corresponding gene
SEQ ID NO:34: Designed oligonucleotide primer for amplifying DNA fragment of corresponding gene
SEQ ID NO:35: Designed oligonucleotide primer for amplifying DNA fragment of corresponding gene
SEQ ID NO:36: Designed oligonucleotide primer for amplifying DNA fragment of corresponding gene
SEQ ID NO:37: Designed oligonucleotide primer for amplifying DNA fragment of corresponding gene
SEQ ID NO:38: Designed oligonucleotide primer for amplifying DNA fragment of corresponding gene
SEQ ID NO:39: Designed oligonucleotide primer for amplifying DNA fragment of corresponding gene
SEQ ID NO:40: Designed oligonucleotide primer for amplifying DNA fragment of corresponding gene

## Claims

1. A substrate for immobilizing ligands coated with an activated polyanion which has been activated beforehand.

2. The substrate according to claim 1, wherein the activated polyanion is an activated carboxylate having a functional group susceptible to substitution or elimination.

3. The substrate according to claim 2, wherein the activated carboxylate is polyacrylate succinimide ester.

4. A method for preparing a substrate for immobilizing ligands, the method comprising activating a polyanion into an activated polyanion on a polyanion-coated substrate for immobilizing ligands.

5. The method according to claim 4, wherein the activated polyanion is an activated carboxylate having a functional group susceptible to substitution or elimination.

6. The method according to claim 5, wherein the activated carboxylate is polyacrylate succinimide ester.

7. A material having immobilized ligands in which nucleic acids are immobilized in predetermined regions on a surface of the substrate for immobilizing ligands defined by claim 1.
